(19) Europäisches Patentamt

European Patent Office

Office européen des brevets

(11) EP 0 838 431 B1

(12) **EUROPÄISCHE PATENTSCHRIFT**

(45) Veröffentlichungstag und Bekanntmachung des Hinweises auf die Patenterteilung:
**10.05.2000 Patentblatt 2000/19**

(51) Int Cl.[7]: **C01B 37/00**, B01J 29/03, C07D 301/12

(21) Anmeldenummer: **97118009.6**

(22) Anmeldetag: **17.10.1997**

(54) **Verfahren zur Herstellung von titanhaltigen Molekularsieben**

Process for the preparation of titanium-containing molecular sieves

Procédé de préparation de tamis moléculaires contenant du titane

(84) Benannte Vertragsstaaten:
**BE DE ES FR IT NL SE**

(30) Priorität: **25.10.1996 DE 19644348**

(43) Veröffentlichungstag der Anmeldung:
**29.04.1998 Patentblatt 1998/18**

(73) Patentinhaber: **Degussa-Hüls Aktiengesellschaft 60287 Frankfurt am Main (DE)**

(72) Erfinder:
• **Hasenzahl, Steffen, Dr. 63457 Hanau (DE)**
• **Thiele, Georg, Dr. 63452 Hanau (DE)**

(56) Entgegenhaltungen:
**EP-A- 0 292 363       EP-A- 0 659 685
FR-A- 2 471 950**

• **ZHANG GUANGYU ET AL.: "Preparation of colloidal suspensions of discrete TS-1 crystals" CHEMISTRY OF MATERIALS., Bd. 9, Nr. 1, Januar 1997, WASHINGTON US, Seiten 210-217, XP000683976**
• **SUDHAKAR REDDY J. ET AL.: "Synthesis, characterization and catalytic properties of titanium silicate, TS-2, with MEL structure" JOURNAL OF CATALYSIS., Bd. 130, Nr. 2, August 1991, MN US, Seiten 440-446, XP002009961**

## Beschreibung

Die vorliegende Erfindung betrifft ein Verfahren zur Herstellung von kristallinen titanhaltigen Molekularsieben der Zusammensetzung $(SiO_2)_{1-x} (TiO_2)_x$ ($0,001 \leq x \leq 0,167$), ausgehend von einem Tetraalkylorthosilicat, einem Tetraalkylorthotitanat und einer wäßrigen Templatlösung.

Bekannt sind kristalline Molekularsiebe der Zusammensetzung $(SiO_2)_{1-x} (TiO_2)_x$, in denen Titanatome einen Teil der Siliciumatome im Kristallgitter ersetzen. Im einzelnen sind dies Titansilicalit-1 mit MFI-Kristallstruktur (DE 30 47 798); Titansilicalit-2 mit MEL-Kristallstruktur (BE 1 001 038 und J.S. Reddy, R. Kumar, P. Ratnasamy, Appl. Catal. 58 (1990) L1; Titan-Beta-Zeolith mit BEA-Kristallstruktur(M.A. Camblor, A. Corma, A. Martines, I. Perez-Pariente, J. Chem. Soc., Chem. Commun. 1992, 589 und EP 659 685); TS-48 mit der Kristallstruktur von Zeolith ZSM48 (D.P. Serrano, H.X. Li, M.E. Davis, J. Chem. Soc., Chem. Commun. 1992, 745).

Die bekannten titanhaltigen kristallinen Molekularsiebe finden Anwendung als Katalysatoren, vor allem für Oxidationsreaktionen mit Wasserstoffperoxid oder organischen Hydroperoxiden. Beispiele sind die Verwendung von Titansilicat-1 als Katalysator zur Umsetzung von Olefinen mit Wasserstoffperoxid zu Epoxiden (EP 100 119); zur Umsetzung von Aromaten mit Wasserstoffperoxid zu Hydroxyaromaten (DE 31 35 559); zur Umsetzung von aliphatischen Kohlenwasserstoffen mit Wasserstoffperoxid zu Alkoholen und Ketonen (EP 376 453) und die Umsetzung von Cyclohexanon mit Wasserstoffperoxid und Ammoniak zu Cyclohexanonoxim (EP 208 311).

Beispiele für die Verwendung von Titansilicat-2 als Katalysator zur Hydroxylierung von Phenol (J.S. Reddy, S. Sivasanker, P. Ratnasamy, J. Mol. Catal. 71 (1992) 373) und zur Umsetzung von Cyclohexanon mit Wasserstoffperoxid und Ammoniak zu Cyclohexanonoxim eingesetzt sind bekannt (J.S. Reddy, S. Sivasanker, P. Ratnasamy, J. Mol. Catal. 71 (1992) 383). Titan-Beta-Zeolith kann als Katalysator zur Umsetzung von Olefinen mit Wasserstoffperoxid oder organischen Hydroperoxiden zu Epoxiden eingesetzt werden( A. Corma, P. Esteve, A. Martines, S. Valencia, J. Catal. 152 (1995) 18 und EP 659 685).

Die bekannten titanhaltigen kristallinen Molekularsiebe werden durch Hydrothermalsynthese hergestellt. Dabei werden zunächst ein Tetraalkylorthosilicat als Siliciumquelle und ein Tetraalkylorthotitanat als Titanquelle mit einem tetrasubstituierten Ammoniumhydroxid als basischer und strukturbestimmender Komponente (Templat) in Gegenwart von Wasser hydrolisiert, danach der aus der Hydrolyse des Tetraalkylorthosilicats und des Tetraalkylorthotitanats gebildeten Alkohol abdestilliert, das entstandene Sol bei einer Temperatur von über 100 °C unter Druck kristallisiert, der gebildete Feststoff abgetrennt, gewaschen, getrocknet und bei einer Temperatur von über 300 °C kalziniert.

Es ist weiterhin bekannt, Titansilicalit-1 ausgehend von Tetraethylorthosilicat und Tetraethylorthotitanat herzustellen. Dabei wird zur Herstellung des Synthesesols ein Gemisch von Tetraethylorthosilicat und Tetraethylorthotitanat vorgelegt und durch Zugabe einer wäßrigen Tetrapropylammoniumhydroxidlösung hydrolisiert. Der dabei entstehende Alkohol wird anschließend abdestilliert und das resultierende Sol bei einer Temperatur von 175 °C unter autogenem Druck zur Kristallisation gebracht (US-Patent 4,410,501).

Aus EP 543 247 ist bekannt, daß bei der Synthese von Titansilicalit-1 anstelle von Tetrapropylammoniumhydroxid auch eine Kombination von Tetra-n-propylammoniumbromid und Ammoniak eingesetzt werden kann.

Zur Herstellung von Titansilicalit-2 (MEL-Struktur) wird Tetra-n-butylammoniumhydroxid und zur Herstellung von Titan-Beta-Zeolith (BEA-Struktur) Tetraethylammoniumhydroxid eingesetzt.

Nach B. Notari, Stud. Surf. Sci. Catal. 67 (1991) 243 ist die Hydrolyse des Gemisches von Silicium- und Titankomponente mit der wäßrigen Lösung des basischen Templat ein kritischer und störanfälliger Schritt. So muß die Bildung eines titanhaltigen Niederschlags bei der Solpräparation unter allen Umständen vermieden werden, da sonst das Titan für die Kristallisation nicht zur Verfügung steht und Materialien geringerer katalytischer Aktivität, die außerdem mit Titandioxid verunreinigt sind, entstehen.

Nach A.J.H.P. van der Pol et al., Appl. Catal. A. 92 (1992) 93 - 111 kann die Bildung eines schwerlöslichen Niederschlages bei der Herstellung eines Synthesesols für die Titansilicalitsynthese vermieden werden, wenn das Gemisch von Tetraethylorthosilicat und Tetraethylorthotitanat zunächst auf 0 °C abgekühlt wird, und die ebenfalls auf 0 °C vorgekühlte wäßrige Tetrapropylammoniumhydroxidlösung unter Rühren tropfenweise zudosiert wird. Dies gilt entsprechend für die Synthese von Titansilicalit-2 und Titan-Beta-Zeolith.

Die bekannten Verfahren haben den Nachteil, daß die Herstellung des Synthesesols aufwendig aud störanfällig ist.So nimmt die Zudosierung der wäßrigen Templatlösung mehrere Stunden in Anspruch, wobei Temperatur und Zugabegeschwindigkeit exakt eingehalten werden müssen. Außerdem ist ein zusätzlicher Arbeitsschritt zum Abdestillieren des bei der Hydrolyse entstandenen Alkohols notwendig.

Aufgabe der Erfindung ist es, ein Verfahren der Synthese von titanhaltigen Molekularsieben zu entwickeln, das sich insbesondere durch eine einfache Solherstellung auszeichnet.

Gegenstand der Erfindung ist ein Verfahren zur Herstellung von kristallinen titanhaltigen Molekularsieben der Zusammensetzung $(SiO_2)_{1-x}(TiO_2)_x$ ($0,001 \leq x \leq 0,167$), welches dadurch gekennzeichnet ist, daß man ein Gemisch aus einem Tetraalkylorthosilikat und einem Tetraalkylorthotitanat vorlegt, eine wäßrige Templatlösung zugibt, die resultierende Reaktionsmischung, die ein Syntheselgel enthält, ohne Abdestillation der bei der Hydrolyse entstandenen Alkohole, in einen Autoklaven gibt und bei einer Temperatur von mehr als 100 °C, vorzugsweise bei 170 bis 190 °C, unter autogenem Druck kristallisiert, anschließend den erhaltenen Feststoff auf bekanntem Wege von der Reaktionsmischung abtrennt, gegebenenfalls wäscht, und bei 400 bis 1000 °C kalziniert, wobei die Templat-Verbindung eine Tetra-n-propylammoniumverbindung ist und ein Titan enthaltendes Molekularsieb mit MFI-Struktur gebildet wird oder die Templat-Verbindung eine Tetra-n-butylammoniumverbindung ist und ein Titan enthaltendes Molekularsieb mit MEL-Struktur erhalten wird.

Zur Herstellung von titanhaltigen Molekularsieben werden bevorzugt solche Tetraalkylorthosilicate und Tetraalkylorthotitanate eingesetzt, bei den Alkyl für Methyl, Ethyl, Propyl oder Butyl, vorzugsweise Ethyl steht.

Die Templatverbindungen werden vorzugsweise als wäßrige Lösungen eingesetzt.

Der für die Synthese erfoderliche pH-Wert des Synthesesols von > 9, bevorzugt > 11, kann wahlweise durch die Verwendung von ausreichenden Mengen eines basisch reagierenden Templats cder durch den Zusatz einer Base, wie Ammoniak oder einem organischen Amin, wie zum Beispiel Methylamin oder 1,6-Hexamethylendiamin erreicht werden.

Die Mengenverhältnisse der Ausgangsstoffe können im Bereich der Molvertältnisse

$(RO)_4$ Si/$(RO)_4$ Ti = 5-1000, bevorzugt 35-60
Templat/$(RO)_4$ Si = 0,04-2,0, bevorzugt 0,2-0,4
OH$^-$/$(RO)_4$ Si = 0,04-2,0, bevorzugt 0,2-0,4
$H_2O$/$(RO)_4$ Si = 5-200, bevorzugt 15-30

gewählt werden, wobei R für eine Alkylgruppe aus der Reihe Methyl, Ethyl, Propyl oder Butyl steht.

Die Temperatur, bei der das Synthesesol hergestellt wird, kann in weiten Grenzen gewählt werden. Bevorzugt wird die Herstellung bei Raumtemperatur durchgeführt. Auch die Zugabegeschwindigkeit der wäßrigen Templatlösung kann in einem weiten Bereich variiert werden. Der Ökonomie der Synthese wegen wird jedoch eine Zugabe innerhalb weniger Minuten bevorzugt.

Das Synthesegel wird anschließend, gegebenenfalls nach einer zusätzlichen Reifezeit, bei einer Temperatur von mehr als 100 °C, vorzugsweise bei 170 bis 190 °C, kristallisiert, wobei die Kristallisationszeit je nach der angewandten Temperatur zwischen 0,5 h und 14 Tagen betragen kann. Eine Kristallisationsdauer von 24h ist im allgemeinen ausreichend. Die Kristalle werden durch Filtrieren, Zentrifugieren oder Dekantieren von der Mutterlauge abgetrennt und mit einer geeigneten Waschflüssigkeit, vorzugsweise Wasser, gewaschen. Anschließend werden die Kristalle gegebenenfalls getrocknet und zur Entfernung des Templats bei einer Temperatur zwischen 400 und 1000 °C, vorzugsweise zwischen 500 und 750 °C, kalziniert.

Zur Entfernung von eventuell enthaltenden Alkaliionen kann man das kalzinierte Produkt wahlweise noch einmal mit einer Lösung eines Ammoniumsalzes, vorzugsweise einer wäßrigen Lösung von Ammoniumnitrat oder Ammoniumacetat, oder mit einer starken Mineralsäure, vorzugsweise einer wäßrigen Lösung von Schwefelsäure, Salzsäure oder Salpetersäure, behandeln und erneut kalzinieren.

Die bei der Kristallisation verbleibende Mutterlauge kann nach vorherigem Abdestillieren des Alkohols ganz oder teilweise in das erfindungsgemäße Verfahren zurückgeführt werden, indem sie bei der Herstellung des Synthesegels anstelle von Wasser eingesetzt wird. Die zur Herstellung des Synthesegels benötigte Menge an Templat reduziert sich dann um die Menge, die nach der Kristallisation in der Mutterlauge verbleibt.

Mit dem erfindungsgemäßen Verfahren können kristalline titanhaltige Molekularsiebe, die sich besonders als Katalysatoren für Oxidationsreaktionen mit Wasserstoffperoxid oder organischen Hydroperoxiden eignen, hergestellt werden.

Die erfindungsgemäßen titanhaltigen Molekularsiebe können vorzugsweise bei folgenden Reaktionen eingesetzt werden:

1. Die Epoxidierung von Olefinen mit Wasserstoffperoxid, wobei als Olefin beispielsweise Propen, 1-Buten, cis- und trans-2-Buten, 1-Penten, 1-Hexen, 1-Octen, 1-Decen, 1-Dodecen, Isobuten, Isoamylen, Butadien, Isopren, Cyclopenten, Cyclohexen, Cycloocten, Allylchlorid, Allylalkohol oder Allylacetat eingesetzt werden können.

2. Die Hydroxylierung von Aromaten mit Wasserstoffperoxid, beispielsweise von Benzol, Toluol, den Xylolen, Phenol oder Anisol.

3. Die Ammoximierung von Carbonylverbindungen mit Wasserstoffperoxid und Ammoniak zu den entsprechenden Oximen, wobei als Carbonylverbindung beispielsweise Aceton, 2-Butanon, Cyclohexanon, Cyclododecanon, Benzaldehyd oder Acetophenon eingesetzt werden können.

4. Die Oxidation von sekundären Alkoholen mit Wasserstoffperoxid zu Ketonen.

**EP 0 838 431 B1**

Die erfindungsgemäßen kristallinen, titanhaltigen Molekularsiebe werden in Pulverform erhalten. Für ihre Anwendung als Oxidationskatalysatoren können sie wahlweise durch bekannte Verfahren zur Verformung von Pulverkatalysatoren, wie beispielsweise Granulation, Sprühtrocknung, Sprühgranulation oder Extrusion in eine zur Anwendung geeignete Form, wie beispielsweise Mikrogranulate, Kugeln, Tabletten, Vollzylinder, Hohlzylinder oder Wabenkörper, gebracht werden.

Der erfindungsgemäß hergestellte titanhaltige Molekularsieb kann als Katalysator bei Oxidationsreaktionen mit $H_2O_2$ eingesetzt werden. Insbesondere kann der erfindungsgemäße titanhaltige Molekularsieb als Katalysator bei der Epoxidierung von Olefinen, vorzugsweise bei der Epoxidierung von Propen mittels $H_2O_2$, eingesetzt werden.

Das erfindungsgemäße Verfahren hat den Vorteil, daß insbesondere der Schritt der Solherstellung einfacher und weniger störanfällig als bei den bekannten Verfahren ist.

**Beispiele**

**Beispiel 1 (gemäß Erfindung)**

In einem Polyethylenbecherglas werden 91,7 g Tetraethylorthosilicat vorgelegt und mit Argon überschichtet. Dann werden unter Rühren 2,88 g Tetraethylorthotitanat tropfenweise zugegeben. Die Reaktionsmischung wird auf 35 °C erwärmt und bei dieser Temperatur eine halbe Stunde gerührt. Anschließend läßt man sie wieder auf Raumtemperatur abkühlen und gibt unter Rühren eine Mischung von 80,5 g Tetraethylammoniumhydroxidlösung (40 Gew.-%, wäßrige Lösung) und 175,0 g entionisiertes Wasser innerhalb von fünf Minuten zu. Die resultierende milchig-weiße Reaktionsmischung wird in einen Autoklaven gegeben und unter Rühren 24 h bei 175 °C kristallisiert.

Der erhaltene Feststoff wird durch Zentrifugieren von der Mutterlauge abgetrennt, dreimal mit entionisiertem Wasser gewaschen, bei 90 °C über Nacht getrocknet und in Luftatmosphäre fünf Stunden bei 500 °C kalziniert. Anschließend wird das Produkt zwei Stunden mit einer 1,0 N Ammoniumacetatlösung bei 80 °C behandelt, mit entionisiertem Wasser gewaschen, getrocknet und erneut bei 500 °C kalziniert.

Mittels chemischer Analyse bestimmter Titangehalt: 2.9
Gew.-% $TiO_2$ Röntgendiffraktogramm:         MFI-Struktur
IR-Spektrum:         Bande bei 960 cm$^{-1}$

**Beispiel 2 (Vergleichsbeispiel)**

Titansilicalit-1 wird in Anlehnung an A.J.H.P. van der Pol et al., Appl. Catal. A 92 (1992) 93-111 hergestellt. Dazu werden 208,5 g Tetraethylorthosilicat in einem Polyethylen-Becherglas vorgelegt und mit Argon überschichtet. Dann werden unter Rühren 6,54 g Tetraethylorthotitanat innerhalb von fünf Minuten zugetropft. Die Reaktionsmischung wird auf 35 °C erwärmt und bei dieser Temperatur für eine halbe Stunde gerührt. Anschließend wird sie abgekühlt und bei 0°C eine Mischung von 183,0 g Tetrapropylammoniumhydroxidlöschung (40 Gew.-%) und 397,8 g entionisiertem Wasser innerhalb von fünf Stunden zugetropft. Danach wird die Reaktionsmischung innerhalb von einer Stunde auf 80 °C erhitzt und bei dieser Temperatur für etwa vier Stunden gehalten, um die Hydrolyse zu vervollständigen und den gebildeten Ethanol abzudestillieren. Durch Zugabe von entionisiertem Wasser wird das Volumen konstant gehalten. Das resultierende wasserklare Sol wird in einen Autoklaven gegeben und unter Rühren 48 h bei 175 °C kristallisiert. Der erhaltene Feststoff wird durch Zentrifugieren von der Mutterlauge abgetrennt, dreimal mit entionisiertem Wasser gewaschen, bei 90 °C über Nacht getrocknet und in Luftatmosphäre vier Stunden bei 500 °C kalziniert. Anschließend wird das Produkt zwei Stunden mit einer 1,0 N Ammoniumacetatlösung bei 80 °C behandelt, mit entionisiertem Wasser gewaschen, getrocknet und erneut bei 500 °C kalziniert.

Mittels chemischer Analyse bestimmter

Titangehalt:         2.4 Gew.-% $TiO_2$.
Röntgendiffraktogramm:         MFI-Struktur.
IR-Spektrum:         Bande bei 960 cm$^{-1}$.

Anwendungsbeispiele zur Epoxidierung von Propylen mit Wasserstoffperoxid.

**Beispiel 3**

In einem thermostatisierten Laborautoklaven mit Begasungsrührer werden 1,0 g des gemäß Beispiel 1 hergestellten Katalysators in 300 ml Methanol bei 40 °C unter Propylenatmosphäre vorgelegt und das Lösungsmittel bei 3 bar Überdruck mit Propylen gesättigt. Dann werden unter Rühren 13,1 g 30 Gew.-% wäßrige Wasserstoffperoxidlösung in einer Portion zugegeben und die Reaktionsmischung bei 40 °C und 3 bar gehalten, wobei über einen Druckregler Propylen nachdosiert wird, um den Verbrauch durch die Reaktion auszugleichen. In regelmäßigen Abständen werden über ein Filter Proben entnommen und der Wasserstoffperoxidgehalt der Reaktionsmischung durch Redoxitration mit Cer(IV)sulfat-Lösung bestimmt. Die Auftragung von $\ln(c/c_0)$ gegen die Zeit t, wobei c die gemessene $H_2O_2$-Konzentration zum Zeitpunkt t und Co die berechnete $H_2O_2$-Konzentration zu Beginn der Reaktion ist, ergibt eine Gerade. Aus der Steigung der Geraden wird mit der Beziehung $\frac{dc}{dt} = -k \cdot c \cdot c_{kat}$, worin $c_{kat}$ für die Katalysatorkonzentration in kg Katalysator je kg Reaktionsmischung steht, die Aktivitätskennzahl k zu 24,4 $min^{-1}$ bestimmt.

**Beispiel 4**

Beispiel 3 wird mit dem in Beispiel 2 hergestellten Katalysator wiederholt. Die Aktivitätskennzahl wird zu 17,2 $min^{-1}$ bestimmt.

**Patentansprüche**

1. Verfahren zur Herstellung von kristallinen, titanhaltigen Molekularsieben der Zusammensetzung $(SiO_2)_{1-x}(TiO_2)_x$ $(0,001 \leq x \leq 0,03)$ mit MFI-Struktur oder MEL-Struktur, dadurch gekennzeichnet, daß man ein Gemisch aus einem Tetraalkylorthosilikat und einem Tetraalkylorthotitanat vorlegt, eine wäßrige Templatlösung zugibt, die resultierende Reaktionsmischung, die ein Synthesegel enthält, ohne Abdestillation der bei der Hydrolyse entstandenen Alkohole, in einen Autoklaven gibt, bei einer Temperatur von mehr als 100 °C, vorzugsweise bei 170 bis 190 °C, unter autogenem Druck kristallisiert, anschließend den erhaltenen Feststoff auf bekanntem Wege von der Reaktionsmischung abtrennt, gegebenenfalls wäscht und bei einer Temperatur von 400 bis 1000 °C kalziniert, wobei die Templat-Verbindung eine Tetra-n-propylammonium-verbindung ist und ein Titan enthaltendes Molekularsieb mit MFI-Struktur gebildet wird oder die Templat-Verbindung eine Tetra-n-butylammoniumVerbindung ist und ein Titan enthaltendes Molekularsieb mit MEL-Struktur erhalten wird.

2. Verfahren nach Anspruch 1, dadurch gekennzeichnet, daß man als Tetraalkylorthosilikat ein Tetraalkylorthosilikat einsetzt, bei dem Alkyl Methyl-, Ethyl-, Propyl- oder Butyl ist.

3. Verfahren nach Anspruch 1, dadurch gekennzeichnet, daß man als Tetraalkylorthotitant ein Tetraalkylorthotitanat einsetzt, bei welchem Alkyl Methyl-, Ethyl-, Propyl- oder Butyl ist.

**Claims**

1. Process for the production of crystalline molecular sieves containing titanium of the composition $(SiO_2)_{1-x}(TiO_2)_x$ $(0.001 \leq x \leq 0.03)$ and having an MFI structure or MEL structure, characterised in that a mixture of a tetraalkyl orthosilicate and a tetraalkyl orthotitanate is initially introduced, an aqueous template solution is added, the resultant reaction mixture, which contains a synthetic gel, is placed in an autoclave, without distilling off the alcohols produced on hydrolysis, is crystallised under autogenic pressure at a temperature of greater than 100°C, preferably at 170 to 190°C, then the resultant solid is separated from the reaction mixture in a known manner, is optionally washed and is calcined at a temperature of 400 to 1000°C, wherein the template compound is a tetra-n-propylammonium compound and a molecular sieve containing titanium and having an MFI structure is formed or the template compound is a tetra-n-butylammonium compound and a molecular sieve containing titanium and having an MEL structure is obtained.

2. Process according to claim 1, characterised in that the tetraalkyl orthosilicate used is a tetraalkyl orthosilicate in which alkyl is methyl, ethyl, propyl or butyl.

3. Process according to claim 1, characterised in that the tetraalkyl orthotitanate used is a tetraalkyl orthotitanate in which alkyl is methyl, ethyl, propyl or butyl.

**Revendications**

1. Procédé de préparation de tamis moléculaires contenant du titane, cristallins, de composition $(SiO_2)_{1-x}(TiO_2)_x$ $(0,001 \leq x \leq 0,03)$ ayant une structure MFI ou une structure MEL,

caractérisé en ce qu'

on utilise un mélange constitué d'un tétraalkylorthosilicate et d'un tétraalkyl-orthotitanate, on ajoute une solution régulatrice aqueuse, on place dans un autoclave le mélange réactif résultant, qui contient un gel de synthèse, sans chasse par distillation des alcools apparus lors de l'hydrolyse, on cristallise sous pression autogène à une température supérieure à 100°C, de préférence à 170 à 190°C, puis on sépare les solides obtenus par des voies connues, hors du mélange réactif, le cas échéant on lave et on calcine à une température de 400 à 1000°C, la liaison régulatrice étant une liaison tétra-n-propylammonium et un tamis moléculaire de structure MFI, contenant du titane, étant constitué, ou la liaison régulatrice est une liaison tétra-n-butylammonium et un tamis moléculaire de structure moléculaire MEL, contenant du titane, étant obtenu.

2. Procédé selon la revendication 1,
   caractérisé en ce qu'
   on utilise comme tétraalkylorthosilicate un tétraalkylorthosilicate pour lequel l'alkyl est un méthyle, éthyle, propyle ou butyle.

3. Procédé selon la revendication 1,
   caractérisé en ce qu'
   on utilise comme tétraalkylorthotitanate un trétraalkylorthotitanate pour lequel l'alkyl est un méthyle, éthyle, propyle ou butyle.